# FASCICULE DE BREVET EUROPEEN

(11) **EP 1 395 230 B1**
(45) Date de publication et mention de la délivrance du brevet: **25.10.2006**
(21) Numéro de dépôt: 02735546.0
(22) Date de dépôt: 21.05.2002
(51) Int. Cl.: A61K 8/06, A61Q 1/10, A61Q 1/02

(54) **COMPOSITION COSMETIQUE FILMOGENE**
FILMBILDENDE KOSMETISCHE ZUSAMMENSETZUNG
FILM-FORMING COSMETIC COMPOSITION

(30) Priorité: 21.05.2001 FR 0106657
(43) Date de publication de la demande: 10.03.2004
(73) Titulaire: L'ORÉAL, 75008 Paris (FR)
(72) Inventeur: COLLIN, Nathalie, F-92330 Sceaux (FR); OLIVIER, Sandrine, F-94240 L'Hay les Roses (FR)
(74) Mandataire: Boulard, Denis
(86) Numéro de dépôt international: PCT/FR2002/001705
(87) Numéro de publication internationale: WO 2002/094206

(56) Documents cités:
- EP-A- 0 557 196
- EP-A- 0 568 035
- US-A- 5 688 493

## Description

La présente invention a pour objet une composition filmogène comprenant un polymère filmogène, une microdispersion aqueuse de cire et un ester d'acide aromatique soluble dans l'eau. Cette composition est utilisable notamment dans le domaine cosmétique pour le maquillage ou le soin des matières kératiniques comme la peau, les cils, les ongles, les cheveux d'être humain.

Plus spécialement, la composition est une composition de maquillage de la peau comme un eye-liner, un fard à paupières, un fard à joues, un produit de maquillage du corps (tatouage semi-permanent).

Les produits de maquillage comme les eye-liners et les fards à paupières sont couramment utilisés pour maquiller les yeux. De plus en plus d'utilisatrices recherchent des produits de maquillage originaux, différents de ceux obtenus avec les poudres de maquillage ou les fards à paupières crèmeux classiques.

Il est connu de la demande EP-A-557 196 un produit de maquillage des yeux comprenant un polymère filmogène hydrosoluble et une microdispersion de cire, qui forme un maquillage brillant. Toutefois, une telle composition s'applique difficilement sur la peau : en effet, le film déposé sur la peau se rétracte lors de l'application et n'adhère pas sur la peau dès son application. Cette composition ne convient donc pas comme eye-liner ou fard à paupières.

Pour améliorer la facilité d'application de la composition, il est possible d'ajouter dans la composition de la glycérine ou du propylène glycol, ou bien encore une silicone polydiméthylsiloxane à greffon polyisobutène et lauryle vendue sous la dénomination « Dow Corning 2-5276 » par la société Dow Corning, mais on a constaté alors que le film déposé sur la peau est trop collant. Or lorsqu'un fard à paupière forme un film trop collant, la partie mobile et la partie fixe de la paupière maquillée se collent entre elles ce qui désagrège le maquillage déposé et confère à l'utilisatrice une sensation très inconfortable.

Pour une bonne application de la composition sur les matières kératiniques, notamment sur la peau, il est également possible d'ajouter un diméthicone copolyol tels que ceux vendus sous les dénominations « ABN SILWER » par la société Nippon Uni Care. Toutefois, cet additif provoque une perte de la brillance du film déposé sur la peau, nuisant ainsi à l'effet de maquillage recherché.

EP-568035 décrit une composition cosmétique contenant un polymère filmogène, une dispersion de cire, un tensioactif et des pigments. Lesdites compositions peuvent comprendre à titre d'additifs des plastifiants choisis dans un liste de composés qui mentionne les esters benzoiques. La composition selon l'invention se différencie donc des compositions de D1 en ce qu'elle comprend une microdispersion aqueuse de cire et par la présence de l'ester d'acide aromatique liquide à température ambiante et soluble dans l'eau.

L'invention a donc pour but dé disposer d'une composition filmogène qui s'applique facilement sur la peau et formant sur celle-ci un film brillant et non collant.

De façon surprenante, les inventeurs ont découvert que l'incorporation d'un ester d'acide aromatique soluble dans l'eau dans une composition comprenant un polymère filmogène et une microdispersion aqueuse de cire permettait une très bonne application, en particulier une facilité d'étalement, de la composition sur les matières kératiniques, notamment sur la peau, sans observer de rétraction du film déposé dès l'application de la composition.

En outre, le film formé suit parfaitement le mouvement de la peau sans se désagréger.

De plus, la composition permet d'obtenir un film très brillant et non collant. Le film obtenu après séchage présente également une bonne tenue sur la peau. Par ailleurs, on peut appliquer sur le film sec un autre produit de maquillage, notamment présentant une couleur différente de celle du film déjà déposé, tout en conservant le contraste des couleurs de chaque produit de maquillage. Les couleurs de chaque produit de maquillage appliqué restent distinctes et ne se mélangent pas, ce qui permet d'obtenir des effets colorés très originaux.

Plus précisément, l'invention a pour objet une composition comprenant, dans un milieu aqueux, un polymère filmogène, une microdispersion aqueuse de cire et un ester d'acide aromatique, liquide à température ambiante et soluble dans l'eau. En particulier, la composition selon l'invention comprend un milieu aqueux physiologiquement acceptable, c'est-à-dire un milieu compatible avec les matières kératiniques comme la peau, tel qu'un milieu cosmétique.

L'invention a également pour objet un procédé cosmétique de maquillage ou de soin non thérapeutique des matières kératiniques, en particulier de la peau, comprenant l'application sur les matières kératiniques, d'une composition telle que définie précédemment.

L'invention a aussi pour objet l'utilisation de la composition telle que définie précédemment pour obtenir un film déposé sur les matières kératiniques, en particulier la peau, brillant et/ou non collant.

L'invention a encore pour objet l'utilisation d'un ester d'acide aromatique, liquide à température ambiante et soluble dans l'eau, d'un polymère filmogène et d'une microdispersion aqueuse de cire, dans une composition cosmétique comprenant un milieu aqueux, pour obtenir une composition qui s'applique et/ou s'étale facilement sur les matières kératiniques, en particulier sur la peau et/ou pour obtenir un film déposé sur les matières kératiniques, en particulier sur la peau, brillant et/ou non collant.

En particulier, la composition selon l'invention peut comprendre un milieu aqueux physiologiquement acceptable, c'est-à-dire comprendre un milieu aqueux compatible avec les matières kératiniques d'êtres humains, comme un milieu aqueux cosmétique.

On entend par microdispersion aqueuse de cire, une dispersion aqueuse de particules de cire, dans laquelle la taille desdites particules de cire est inférieure ou égale à environ 1 µm.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire. Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 1 µm), de préférence inférieures à 0,5 µm (notamment allant de 0,051 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides, à température ambiante d'origine animale, végétale, minérale ou de synthèse et leurs mélanges. De préférence, les cires entrant dans la composition peuvent présenter un point de fusion supérieur à 45°C environ, et en particulier supérieur à 55°C. La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

On peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone, les cires fluorées.

Il est également possible d'utiliser des mélanges commerciaux de cires auto-émulsionnables contenant une cire et des tensioactifs. On peut utiliser par exemple la cire commercialisée sous la dénomination 'Cire Auto Lustrante OFR' par Tiscco, qui contient des cires de Carnauba et de paraffine en association avec des tensioactifs non ioniques, ou la cire auto-émulsionnable commercialisée sous la dénomination 'Cerax A.O. 28/B' par La Ceresine, qui contient de la cire d'Alfa en association avec un tensioactif non ionique. Ces mélanges commerciaux permettent de préparer des microdispersions de cires par simple addition d'eau.
On peut encore citer les produits 'Aquacer' de Byk Cera, et notamment : le mélange de cires synthétiques et naturelles avec émulsionnant anionique (Aquacer 520), la cire de polyéthylène avec émulsionnant non ionique (Aquacer 514 ou 513), la cire polymérique avec émulsionnant anionique (Aquacer 511). On peut également citer le mélange de cires de polyéthylène et de paraffine avec émulsionnant non ionique 'Jonwax 120' de Johnson Polymer.

La cire peut être présente dans la composition selon l'invention en une teneur en matière sèche allant de 0,1 % à 50 % en poids, par rapport au poids total de la composition, de préférence de 0,5 % à 30 % en poids, et mieux de 1 % à 20 % en poids.

La composition peut également comprendre une quantité suffisante de tensioactif pour permettre d'obtenir une microdispersion de cire, ainsi qu'une composition finale, stable. Notamment, elle peut comprendre 0,01 % à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

Il est possible d'introduire en outre dans la phase cireuse microparticulaire des ingrédients actifs liposolubles, tels que des filtres U.V., des vitamines liposolubles, des actifs cosmétiques liposolubles.

Le polymère filmogène présent dans la composition selon l'invention peut être un polymère solubilisé ou dispersé sous forme de particules solides dans la phase aqueuse de la composition. La composition peut comprendre un mélange de ces polymères.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insàturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁₋C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols. L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanédicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ^{®} par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier. Les particules de polymère filmogène peuvent avoir une taille moyenne allant de 5 nm à 600 nm , et de préférence de 20 nm à 300 nm.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEY KOGYO; ou ben encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE-UR-425 ^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   . les muccopolysaccharides tels que l'acide hyaluronique, les chondroïtines sulfate,
   et leurs mélanges.

Par « ester d'acide aromatique soluble dans l'eau », on entend dans la présente demande un ester formé à partir d'acide aromatique et d'un alcool, ledit ester étant soluble dans l'eau à 25 °C à au moins 50 %.

L'ester est liquide à température ambiante, c'est à dire à 25°C.

L'acide aromatique peut être choisi parmi les acides carboxyliques suivants :
a) les mono acides, tels que l'acide benzoïque, l'acide phényl acétique, l'acide cinnamique, l'acide 3 phényl propanoïque, l'acide salicylique ;
b) les diacides tels que l'acide téréphtalique ;
c) les triacides tels que l'acide trimellitique ; et
d) les tétra-acides tels que l'acide pyromellitique.

De façon avantageuse, l'acide carboxylique aromatique est l'acide benzoïque.

Avantageusement, l'ester d'acide aromatique soluble dans l'eau peut résulter de l'estérification par un acide aromatique (tel que défini précédemment) d'au moins un groupe hydroxyle d'un composé hydroxylé choisi parmi les diméthicones copolyol, les polymères blocs polyéthylèneglycol/polypropylèneglycol, les méthyl glucosides polyoxyalkylénés.

Les diméthicones copolyols sont des diméthylpolysiloxane portant une ou plusieurs chaînes latérales de polyoxyéthylène et/ou de polyoxypropylène.

Les polymères blocs polyéthylèneglycol/polypropylèneglycol peuvent être notamment des polycondensats triblocs polyéthylèneglycol/ polypropylèneglycol/polyéthylèneglycol ou polypropylèneglycol/polyéthylèneglycol/ polypropylèneglycol. De tels polymères blocs peuvent avoir un poids moléculaire allant de 1000 à 3300. Ces polymères blocs peuvent comporter de 10 à 50% en poids, par rapport au poids total dudit polymère, de polyéthylèneglycol. De tels polymères blocs sont notamment commercialisés sous la dénomination « PLURONIC^{®}» et « PLURONIC^{®} R » par la société BASF.

Les méthylglucosides polyoxyalkylénés peuvent être des méthylglucosides oxyéthylénés ou oxypropylénés. De tels composés sont notamment commercialisés sous le nom de « LUCAM E » et « GLUCAM P »par la société AMERCHOL.

Selon un premier mode de réalisation de la composition selon l'invention, l'ester d'acide aromatique soluble dans l'eau peut être une diméthicone copolyol benzoate, c'est-à-dire un ester, notamment u esetr partiel, d'acide benzoïque et de diméthicone copolyol, ce dernier étant un polymère de diméthylpolysiloxane comportant des chaînes latérales de polyoxyéthylène et/ou de polyoxypropylène. On entend par ester partiel un composé dont une partie des groupes hydroxyles sont estérifiés. Comme diméthicone copolyol benzoate, on peut utiliser ceux vendus sous la dénomination « FINSOLV^{®} SLB-101 », « FINSOLV^{®} SLB-201 » par la société FINETEX.

Selon un deuxième mode de réalisation de la composition selon l'invention, l'ester d'acide aromatique soluble dans l'eau peut être un benzoate de copolymère bloc polyéthylèneglycol/polypropylèneglycol, notamment un benzoate de polycondensats triblocs polyéthylèneglycol/ polypropylèneglycol/polyéthylèneglycol ou polypropylèneglycol/polyéthylèneglycol/ polypropylèneglycol. De tels benzoates sont décrits dans le brevet US-A-5271930. De tels composés répondent notamment aux formules (III) et (IV) suivantes :

R⁵-CO-O-(-CH₂-CH₂-O)ₓ-(-CH₂-CH(CH₃)-O)_{y}-(CH₂-CH₂-O)ₓ-O-CO-R'⁵ (III)

R⁵-CO-O-(-CH(CH₃)-CH₂-O)ₓ-(-CH₂- CH₂-O)_{y}-(CH₂-CH(CH₃)-O)ₓ-O-CO-R'⁵ (IV)

dans lesquelles :
- R⁵ et R'⁵, désignent, indépendamment l'un de l'autre, un atome d'hydrogène ou un radical phényl, au moins l'un des radicaux R⁵ et R'⁵ désignant un radical phényl,
- x et y désignant, indépendamment l'un de l'autre, un nombre allant de 2 à 100, de préférence allant dé 2 à 30.

Comme benzoate de copolymère bloc polyéthylèneglycol/polypropylèneglycol, on peut utiliser ceux vendus sous les dénomination « FINSOLV^{®} PL-62 », « FINSOLV^{®} PL-355 » par la société FINETEX.

Selon un troisième mode de réalisation de la composition selon l'invention, l'ester d'acide aromatique soluble dans l'eau peut être un benzoate de méthylglucoside oxyéthyléné ou oxypropyléné, tel que ceux décrits dans le brevet US-A-5270461. De tels composés répondent notamment aux formules (I) et (II) suivantes : 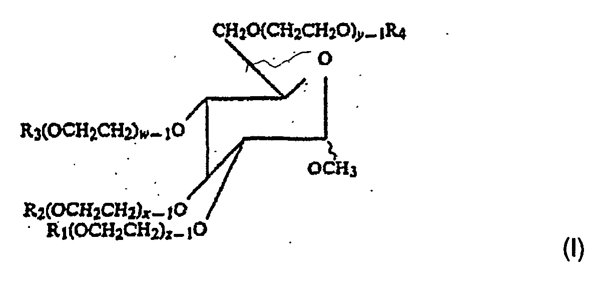 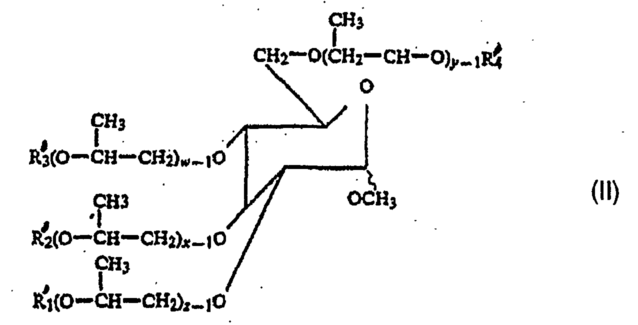 dans lesquelles :
- R₁, R₂, R₃, R₄ désignent 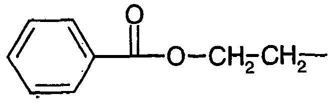
- R'₁, R'₂, R'₃, R'₄ désignent 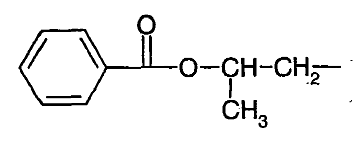 et w+x+y+z va de 10 à 20.

Comme benzoate d'alcoxyméthyl glucoside, on peut utiliser celui vendu sous la dénomination commerciale « FINSOLV^{®} EMG-20 » par la société FINETEX.

De préférence, l'ester d'acide aromatique soluble dans l'eau est choisi parmi les diméthicone copolyol benzoate et les benzoates de copolymère bloc polyéthylèneglycol/polypropylèneglycol définis précédemment.

Avantageusement, l'ester d'acide aromatique soluble dans l'eau n'est pas un benzoate de méthylglucose oxyéthyléné à 20 motifs d'oxyde d'éhylène.

L'ester d'acide aromatique soluble dans l'eau peut être présent dans la composition selon l'invention en une teneur allant de 0,1 % à 20 % en poids, par rapport au poids total de la composition, de préférence allant de 1 % à 15 % en poids, et mieux allant de 5 % à 15 % en poids.

Avantageusement, le polymère filmogène et l'ester d'acide aromatique peuvent être présents dans la composition selon un rapport pondéral polymère filmogène/ester d'acide aromatique allant de 0,1 à 3, et de préférence allant de 0,5 à 2,5.

Le milieu aqueux de la composition peut être constitué essentiellement d'eau. Ile peut comprendre également un mélange d'eau et de solvant miscible à l'eau comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄. Le mileiu aqueux (eau et éventuellement le solvant organique miscible à l'eau) peut représenter, en pratique, de 5 % à 90 % en poids, par rapport au poids total de la composition.

La composition peut également comprendre au moins une matière colorante comme les composés pulvérulents, par exemple à raison de 0,01 à 50 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet dé manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les oligo-éléments, les adoucissants, les séquestrants, les parfums, les huiles, les silicones, les épaississants, les vitamines, les protéines, les céramides, les plastifiants, les agents de coalescence, les agents de cohésion ainsi que les agents alcalinisants ou acidifiants habituellement utilisés dans le domaine cosmétique, les émollients, les conservateurs.

La composition selon l'invention peut être une composition cosmétique pouvant se présenter sous la forme d'un eye-liner, d'un fard à paupières, d'un produit de maquillage du corps, d'un produit pour les lèvres, d'un mascara, d'un vernis à ongles, d'un fond de teint, d'un produit pour les sourcils, d'un produit capillaire, d'un produit pour le soin de la peau.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemple 1 :

On a préparé une microdispersion de cire de carnauba ayant la composition suivante :
- Cire de carnauba 27 g
- Monostéarate de glycéryle polyoxyéthyléné (30 OE) (TAGAT S de GOLDSCHMIDT) 6,7 g
- Ethanol 10 g
- Eau qsp 100 g

On a chauffé à 95 °C la cire et le tensioactif en homogénéisant le mélange sous agitation modérée. Puis on a incorporé l'eau chauffée à 95 °C en continuant d'agiter. On a refroidit à température ambiante puis ajouté l'éthanol, pour obtenir une microdispersion de cire ayant un diamètre moyen de particules d'environ 170 nm.

### Exemple 2:

On a préparé un eye-liner ayant la composition suivante :
- Microdispersion de cire de l'exemple 1 24 g
- Alcool éthylique 3 g
- Sulfopolyester vendu sous la dénomination
- EASTMAN AQ 55S par la Société EASTMAN CHEMICAL 11 g
- Diméthicone copolyol benzoate vendu sous la dénomination « FINSOLV SLB-101 » par la Société FINETEX 9,5 g
- Tensioactif vendu sous la dénomination "SYMPERONIC^{®} PE/L 44" par la Société ICI 5 g
- Oxyde de fer noir 20 g
- Conservateurs qs
- Eau qsp 100 g

Ce liner s'applique très facilement sur la peau et laisse sur celle-ci, après séchage, un film très brillant, non collant.

### Exemple 3 :

On a préparé un fard à paupières ayant la composition suivante :
- Microdispersion de cire de l'exemple 1 53,7 g
- Ethanol 5,9 g
- Sulfopolyester (AQ 55S d'EASTMAN CHEMICAL) 7,6 g
- Diméthicone copolyol benzoate vendu sous la dénomination « FINSOLV SLB-101 » par la Société FINETEX 9,5 g
- Cyclopentadiméthylsiloxane 3,6 g
- Pigments 4,8 g
- Conservateurs qs
- Eau qsp 100 g

Ce fard à paupières s'étale instantanément sur la paupière et forme après séchage, un maquillage filmogène très brillant, non collant, confortable pour l'utilisatrice.

### Exemple 4 :

On a préparé un fard à paupières ayant la composition suivante :
- Microdispersion de cire de l'exemple 1 33 g
- Ethanol 4,5 g
- Sulfopolyester (AQ 55S d'EASTMAN CHEMICAL) 10,5 g
- Dibenzoate de polyéthylèneglycol (8 OE) oxypropyléné (30 OP) oxyéthyléné (8 OE) vendu sous la dénomination « FINSOLV PL-62 » par la Société FINETEX 12,3 g
- Pigments 10 g
- Conservateurs qs
- Eau qsp 100 g

Ce fard à paupières s'étale instantanément sur la paupière et forme après séchage, un maquillage filmogène brillant et confortable pour l'utilisatrice.

### Exemple 5:

On a préparé un fard à paupières ayant la composition suivante :
- Microdispersion de cire de l'exemple 1 33 g
- Ethanol 4,5 g
- Sulfopolyester (AQ 55S d'EASTMAN CHEMICAL) 10,5 g
- Benzoate de polyéthylèneglycol (11 OE) oxypropyléné (16 OP) oxyéthyléné (11 OE) vendu sous la dénomination « FINSOLV PL-355 » par la Société FINETEX 12,3 g
- Pigments 10 g
- Conservateurs qs
- Eau qsp 100 g

Ce fard à paupières s'étale instantanément sur la paupière et forme après séchage, un maquillage filmogène confortable pour l'utilisatrice.

## Revendications

1. Composition comprenant, dans un milieu aqueux, un polymère filmogène, une microdispersion aqueuse de cire et un ester d'acide aromatique liquide à température ambiante et soluble dans l'eau.

2. Composition selon la revendication 1, **caractérisée par le fait que** la microdispersion de cires comprend des particules de cires ayant une taille moyenne inférieure à 1 µm, de préférence inférieure à 0,5 µm.

3. Composition selon la revendication 1 ou 2, **caractérisée par le fait que** la cire est choisie dans le groupe formé par les cires ayant un point de fusion allant de 30 °C à 120°C.

4. Composition selon l'une quelconque des revendications précédentes, **caractérisée par le fait que** la cire a une dureté allant de 0,05 MPa à 15 MPa.

5. Composition selon l'une des revendications précédentes, **caractérisée par le fait que** la cire est choisie dans le groupe formé par la cire d'abeilles, la cire de lanoline, les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch; les copolymères cireux ainsi que leurs esters; les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

6. Composition selon l'une des revendications précédentes, **caractérisé par le fait que** la cire est présente en une teneur en matière sèche allant de 0,1 % à 50 % en poids, par rapport au poids total dé la composition, de préférence allant de 0,5 % à 30% en poids, et mieux allant de 1 % à 20 % en poids.

7. Composition selon l'une des revendications précédentes, dans laquelle les particules de la dispersion de cire comprennent en outre des additifs gras huileux et/ou pâteux et/ou un additif/actif liposoluble.

8. Composition selon l'une des revendications précédentes, comprenant en outre au moins un tensioactif.

9. Composition selon selon l'une des revendications précédentes, **caractérisée par le fait que** le polymère filmogène est choisi dans le groupe formé par les polymères radicalaires, les polycondensats, les polymères d'origine naturelle et leurs mélanges.

10. Composition selon l'une des revendications précédentes, **caractérisée par le fait que** le polymère filmogène est choisi dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

11. Composition selon l'une des revendications précédentes, **caractérisée par le fait que** le polymère filmogène est choisi dans le groupe formé par les polyuréthanes, les polyesters, les polyesters amides, les polyamides, les résines époxyesters, les polyurées.

12. Composition selon l'une des revendications précédentes, **caractérisée par le fait que** le polymère filmogène est choisi dans le groupe formé par la résine shellac, la gomme de sandaraque, les dammars, les élémis, les copals, les polymères cellulosiques insolubles dans l'eau, et leurs mélanges.

13. Composition selon l'une quelconque des revendications précédentes, **caractérisée par le fait que** le polymère filmogène est sous forme de particules solides dispersées dans la phase aqueuse.

14. Composition selon l'une quelconque des revendications précédentes, **caractérisée par le fait que** le polymère filmogène est présent en une teneur en matière sèche allant de 0,1 % à 60 % en poids, par rapport au poids total de la composition, de préférence allant de 0,5% à 40% en poids, et mieux allant de 1 % à 30 % en poids.

15. Composition selon l'une quelconque des revendications précédentes, **caractérisée par le fait que** l'ester d'acide aromatique soluble dans l'eau résulte de l'estérification par un acide aromatique d'au moins un goupe hydroxyle d'un composé hydroxylé choisi parmi les diméthicones copolyols, les polymères blocs polyéthylèneglycol/polypropylèneglycol, les méthyl glucosides polyoxyalkylénés.

16. Composition selon l'une quelconque des revendications précédentes, **caractérisée par le fait que** l'acide aromatique est choisi dans le groupe formé par l'acide benzoïque, l'acide phényl acétique, l'acide cinnamique, l'acide 3 phényl propanoïque, l'acide salicylique, l'acide téréphtalique, l'acide trimellitique et l'acide pyromellitique.

17. Composition selon l'une quelconque des revendications précédentes, **caractérisée par le fait que** l'acide aromatique est l'acide benzoïque.

18. Composition selon l'une quelconque des revendications précédentes, **caractérisée par le fait que** ledit ester d'acide aromatique est un diméthicone copolyol benzoate.

19. Composition selon la revendication 15, **caractérisée par le fait que** le méthylglucoside polyoxyalkyléné est un méthylglucoside oxyéthyléné ou oxypropyléné.

20. Composition selon l'une quelconque des revendications 1 à 17, **caractérisée par le fait que** ledit ester d'acide aromatique est un benzoate de méthylglucoside oxyéthyléné ou oxypropyléné

21. Composition selon la revendication 20, **caractérisée par le fait que** ledit ester d'acide aromatique soluble dans l'eau est un benzoate de méthylglucoside oxyéthyléné de formule (I) suivante : 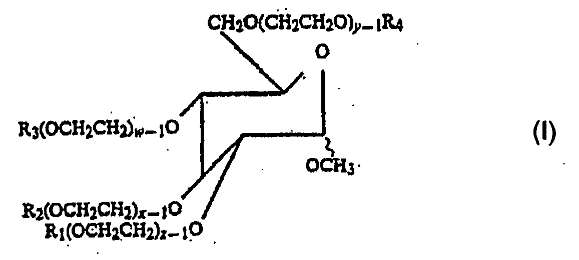 dans laquelle :
- R₁, R₂, R₃, R₄ désignent 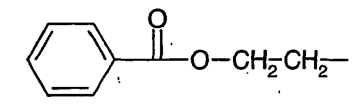 et w+x+y+z va de 10 à 20.

22. Composition selon la revendication 20, **caractérisée par le fait que** ledit ester d'acide aromatique est un benzoate de méthylglucoside oxypropyléné de formule (II) suivante : 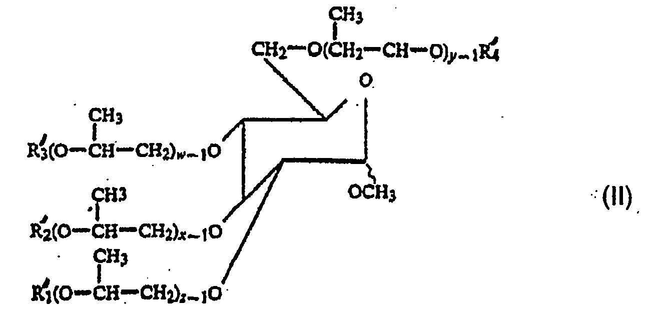 dans laquelle :
- R'₁, R'₂, R'₃, R'₄ désignent 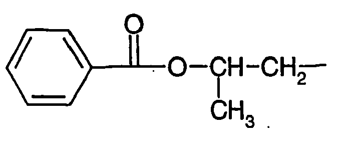 et w + x + y + z va de 10 à 20.

23. Composition selon l'une quelconque des revendications 1 à 17, **caractérisée par le fait que** ledit ester d'acide aromatique résulte de l'estérification d'un acide aromatique avec un polymère bloc polyéthylèneglycol/polypropylèneglycol.

24. Composition selon la revendication 23, **caractérisée par le fait que** polymère bloc polyéthylèneglycol/polypropylèneglycol est choisi parmi les polycondensats triblocs polyéthylèneglycol/ polypropylèneglycol/polyéthylèneglycol ou polypropylèneglycol/polyéthylèneglycol/ polypropylèneglycol.

25. Composition selon la revendication 23 ou 24, **caractérisée par le fait que** ledit ester d'acide aromatique est un benzoate de copolymère bloc polyéthylèneglycol/polypropylèneglycol.

26. Composition selon la revendication 25, **caractérisée par le fait que** ledit ester d'acide aromatique répond à la formule (III) suivante :
R⁵-CO-O-(-CH₂-CH₂-O)ₓ-(-CH₂-CH(CH₃)-O)_{y}-(CH₂-CH₂-O)ₓ-O-CO-R'⁵ (III)
dans laquelle :
- R⁵ et R'⁵, désignent, indépendamment l'un de l'autre, un atome d'hydrogène ou un radical phényl, au moins l'un des radicaux R⁵ et R'⁵ désignant un radical phényl,
- x et y désignant, indépendamment l'un de l'autre, un nombre allant de 2 à 100,

27. Composition selon la revendication 25, **caractérisée par le fait que** ledit ester d'acide aromatique répond à la formule (IV) suivante :
R⁵-CO-O-(-CH(CH₃)-CH₂-O)ₓ-(-CH₂-CH₂-O)_{y}-(CH₂-CH(CH₃)-O)ₓ-O-CO-R'⁵ (IV)
dans laquelle :
- R⁵ et R'⁵, désignent, indépendamment l'un de l'autre, un atome d'hydrogène ou un radical phényl, au moins l'un des radicaux R⁵ et R'⁵ désignant un radical phényl,
- x et y désignant, indépendamment l'un de l'autre, un nombre allant de 2 à 100,

28. Composition selon la revendication 26 ou 27, **caractérisée par le fait que** x et y désigne un nombre allant de 2 à 30.

29. Composition selon l'une quelconque des revendications précédentes, **caractérisée par le fait que** ledit ester d'acide aromatique soluble dans l'eau est présent dans la composition selon l'invention en une teneur allant de 0,1 % à 20 % en poids, par rapport au poids total de la composition, de préférence allant de 1 % à 15 % en poids, et mieux allant de 5 % à 15 % en poids.

30. Composition selon l'une quelconque des revendications précédentes, **caractérisée par le fait que** le polymère filmogène et l'ester d'acide aromatique sont présents selon un rapport pondéral polymère filmogène/ester d'acide aromatique allant de 0,1 à 3, et de préférence allant de 0,5 à 2,5.

31. Composition selon l'une quelconque des revendications précédentes, **caractérisé par le fait que** la composition comprend, en outre, au moins un additif choisi dans le groupe formé par les épaississants, les agents plastifiants, les agents de coalescence, les charges, les matières colorantes, les cires, les tensio-actifs, les conservateurs, les huiles, les parfums.

32. Composition selon l'une quelconque des revendications précédentes, **caractérisée par le fait que** la composition est une composition cosmétique.

33. Composition selon la revendication 32, **caractérisée par le fait qu'** elle se présente sous la forme d'un eye-liner, d'un fard à paupières, d'un produit de maquillage du corps, d'un produit pour les lèvres, d'un mascara, d'un vernis à ongles, d'un fond de teint, d'un produit pour les sourcils, d'un produit capillaire, d'un produit pour le soin de la peau.

34. Procédé cosmétique de traitement non thérapeutique ou de maquillage des matières kératiniques comprenant l'application sur lesdites matières kératiniques d'une composition selon l'une quelconque des revendications 1 à 33.

35. Procédé selon la revendication 34, **caractérisé par le fait que** les matières kératiniques sont la peau.

36. Utilisation d'une composition telle que définie selon l'une quelconque des revendications 1 à 33 pour obtenir un film déposé sur les matières kératiniques brillant et/ou non collant.

37. Utilisation d'un ester d'acide aromatique, liquide à température ambiante et soluble dans l'eau, d'un polymère filmogène et d'une microdispersion aqueuse de cire, dans une composition cosmétique comprenant un milieu aqueux, pour obtenir une composition qui s'applique et/ou s'étale facilement sur les matières kératiniques, en particulier sur la peau, et/ou pour obtenir un film déposé sur les matières kératiniques, en particulier sur la peau, brillant et/ou non collant.

## Claims

1. Composition comprising, in an aqueous medium, a film-forming polymer, an aqueous microdispersion of wax and a water-soluble aromatic acid ester that is liquid at room temperature.

2. Composition according to Claim 1, **characterized in that** the wax microdispersion comprises wax particles with a mean size of less than 1 µm and preferably less than 0.5 µm.

3. Composition according to Claim 1 or 2, **characterized in that** the wax is chosen from the group formed by waxes with a melting point ranging from 30°C to 120°C.

4. Composition according to any one of the preceding claims, **characterized in that** the wax has a hardness ranging from 0.05 MPa to 15 MPa.

5. Composition according to one of the preceding claims, **characterized in that** the wax is chosen from the group formed by beeswax, lanolin wax and Chinese insect waxes; rice wax, carnauba wax, candelilla wax, ouricurry wax, esparto grass wax, cork fibre wax, sugarcane wax, Japan wax and sumach wax; montan wax, microcrystalline waxes, paraffins and ozokerite; polyethylene waxes, the waxes obtained by Fisher-Tropsch synthesis; waxy copolymers and esters thereof; the waxes obtained by catalytic hydrogenation of animal or plant oils containing linear or branched C8-C32 fatty chains; hydrogenated jojoba oil, hydrogenated sunflower oil, hydrogenated castor oil, hydrogenated coconut oil, hydrogenated lanolin oil; silicone waxes; mixtures thereof.

6. Composition according to one of the preceding claims, **characterized in that** the wax is present in a solids content ranging from 0.1% to 50% by weight, preferably ranging from 0.5% to 30% by weight and better still ranging from 1% to 20% by weight relative to the total weight of the composition.

7. Composition according to one of the preceding claims, in which the particles of the wax dispersion also comprise oily and/or pasty fatty additives and/or a liposoluble additive/active agent.

8. Composition according to one of the preceding claims, also comprising at least one surfactant.

9. Composition according to one of the preceding claims, **characterized in that** the film-forming polymer is chosen from the group formed by free-radical polymers, polycondensates and polymers of natural origin, and mixtures thereof.

10. Composition according to one of the preceding claims, **characterized in that** the film-forming polymer is chosen from the group formed by vinyl polymers resulting from the polymerization of monomers chosen from α,β-ethylenic unsaturated carboxylic acids, esters of the said acids, amides of the said acids, vinyl esters and styrene monomers.

11. Composition according to one of the preceding claims, **characterized in that** the film-forming polymer is chosen from the group formed by polyurethanes, polyesters, polyesteramides, polyamides, epoxyester resins and polyureas.

12. Composition according to one of the preceding claims, **characterized in that** the film-forming polymer is chosen from the group formed by shellac resin, sandarac gum, dammar resins, elemi gums, copal resins and water-insoluble cellulose polymers, and mixtures thereof.

13. Composition according to any one of the preceding claims, **characterized in that** the film-forming polymer is in the form of solid particles dispersed in the aqueous phase.

14. Composition according to any one of the preceding claims, **characterized in that** the film-forming polymer is present in a solids content ranging from 0.1% to 60% by weight, preferably ranging from 0.5% to 40% by weight and better still ranging from 1% to 30% by weight relative to the total weight of the composition.

15. Composition according to any one of the preceding claims, **characterized in that** the water-soluble aromatic acid ester results from the esterification with an aromatic acid of at least one hydroxyl group of a hydroxylated compound chosen from dimethicone copolyols, polyethylene glycol/polypropylene glycol block polymers and polyoxyalkylenated methylglucosides.

16. Composition according to any one of the preceding claims, **characterized in that** the aromatic acid is chosen from the group formed by benzoic acid, phenylacetic acid, cinnamic acid, 3-phenylpropanoic acid, salicylic acid, terephthalic acid, trimellitic acid and pyromellitic acid.

17. Composition according to any one of the preceding claims, **characterized in that** the aromatic acid is benzoic acid.

18. Composition according to any one of the preceding claims, **characterized in that** the said aromatic acid ester is a dimethicone copolyol benzoate.

19. Composition according to Claim 15, **characterized in that** the polyoxyalkylenated methylglucoside is an oxyethylenated or oxypropylenated methylglucoside.

20. Composition according to any one of Claims 1 to 17, **characterized in that** the said aromatic acid ester is an oxyethylenated or oxypropylenated methylglucoside benzoate.

21. Composition according to Claim 20, **characterized in that** the said water-soluble aromatic acid ester is an oxyethylenated methylglucoside benzoate of formula (I) below: 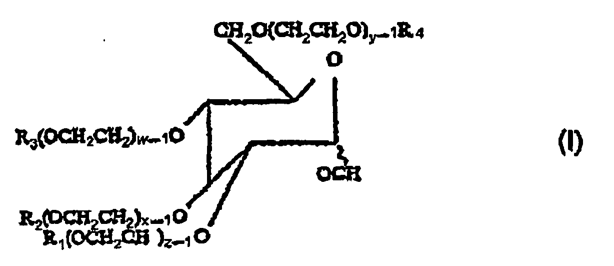 in which:
- R₁, R₂, R₃ and R₄ denote 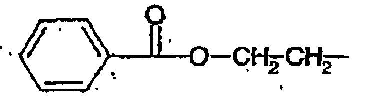 and w + x + y + z ranges from 10 to 20.

22. Composition according to Claim 20, **characterized in that** the said aromatic acid ester is an oxypropylenated methylglucoside benzoate of formula (II) below: 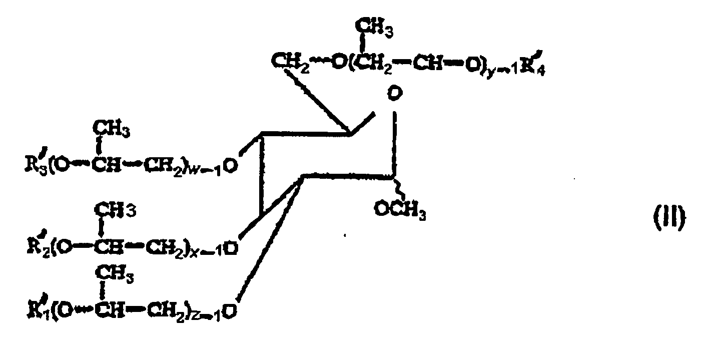 in which:
- R'₁, R'₂, R'₃ and R'₄ denote 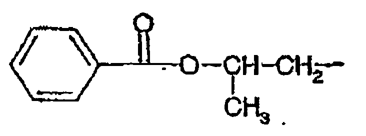 and w + x + y + z ranges from 10 to 20.

23. Composition according to any one of Claims 1 to 17, **characterized in that** the said aromatic acid ester results from the esterification of an aromatic acid with a polyethylene glycol/polypropylene glycol block polymer.

24. Composition according to Claim 23, **characterized in that** the polyethylene glycol/polypropylene glycol block polymer is chosen from polyethylene glycol/polypropylene glycol/polyethylene glycol or polypropylene glycol/polyethylene glycol/polypropylene glycol triblock polycondensates.

25. Composition according to Claim 23 or 24, **characterized in that** the said aromatic acid ester is a benzoate of polyethylene glycol/polypropylene glycol block copolymer.

26. Composition according to Claim 25, **characterized in that** the said aromatic acid ester corresponds to formula (III) below:
R⁵-CO-O-(-CH₂-CH₂-O)ₓ-(-CH₂-CH(CH₃)-O)_{y}-(CH₂-CH₂-O)ₓ-O-CO-R'⁵ (III)
in which:
- R⁵ and R'⁵ denote, independently of each other, a hydrogen atom or a phenyl radical, at least one of the radicals R⁵ and R'⁵ denoting a phenyl radical,
- x and y denoting, independently of each other, a number ranging from 2 to 100.

27. Composition according to Claim 25, **characterized in that** the said aromatic acid ester corresponds to formula (IV) below:
R⁵-CO-O-(-CH(CH₃)-CH₂-O)ₓ-(-CH₂-CH₂-O)_{y}-(CH₂-CH(CH₃)-O)ₓ-O-CO-R'⁵ (IV)
in which:
- R⁵ and R'⁵ denote, independently of each other, a hydrogen atom or a phenyl radical, at least one of the radicals R⁵ and R'⁵ denoting a phenyl radical,
- x and y denoting, independently of each other, a number ranging from 2 to 100.

28. Composition according to Claim 26 or 27, **characterized in that** x and y denote a number ranging from 2 to 30.

29. Composition according to any one of the preceding claims, **characterized in that** the said water-soluble aromatic acid ester is present in the composition according to the invention in a content ranging from 0.1% to 20% by weight, preferably ranging from 1% to 15% by weight and better still ranging from 5% to 15% by weight relative to the total weight of the composition.

30. Composition according to any one of the preceding claims, **characterized in that** the film-forming polymer and the aromatic acid ester are present in a film-forming polymer/aromatic acid ester weight ratio ranging from 0.1 to 3 and preferably ranging from 0.5 to 2.5.

31. Composition according to any one of the preceding claims, **characterized in that** the composition also comprises at least one additive chosen from the group formed by thickeners, plasticizers, coalescers, fillers, colorants, waxes, surfactants, preserving agents, oils and fragrances.

32. Composition according to any one of the preceding claims, **characterized in that** the composition is a cosmetic composition.

33. Composition according to Claim 32, **characterized in that** it is in the form of an eyeliner, an eyeshadow, a body makeup product, a lip product, a mascara, a nail varnish, a foundation, an eyebrow product, a haircare product, or a skincare product.

34. Cosmetic process for the non-therapeutic treatment of or for making up keratin materials, comprising the application to the said keratin materials of a composition according to any one of Claims 1 to 33.

35. Process according to Claim 34, **characterized in that** the keratin materials are the skin.

36. Use of a composition as defined according to any one of Claims 1 to 33, to obtain a shiny and/or non-sticky film deposited on keratin materials.

37. Use of a water-soluble aromatic acid ester that is liquid at room temperature, of a film-forming polymer and of an aqueous microdispersion of wax, in a cosmetic composition comprising an aqueous medium, to obtain a composition that applies and/or spreads easily onto keratin materials, in particular the skin, and/or to obtain a shiny and/or non-sticky film deposited on keratin materials, in particular on the skin.

## Patentansprüche

1. Zusammensetzung, die in einem wässrigen Medium ein filmbildendes Polymer, eine wässrige Wachsmikrodispersion und einen bei Umgebungstemperatur flüssigen und wasserlöslichen Ester einer aromatischen Säure enthält.

2. Zusammensetzung nach Anspruch 1, **dadurch gekennzeichnet, dass** die Mikrodispersion von Wachsen Partikel von Wachsen enthält, die eine mittlere Größe unter 1 µm und vorzugsweise unter 0,5 µm aufweisen.

3. Zusammensetzung nach Anspruch 1 oder 2, **dadurch gekennzeichnet, dass** das Wachs unter den Wachsen mit einem Schmelzpunkt von 30 bis 120 ° C ausgewählt ist.

4. Zusammensetzung nach einem der vorhergehenden Ansprüche, **dadurch gekennzeichnet, dass** das Wachs eine Härte von 0,05 bis 15 MPa besitzt.

5. Zusammensetzung nach einem der vorhergehenden Ansprüche, **dadurch gekennzeichnet, dass** das Wachs unter Bienenwachs, Lanolinwachs, Chinawachs; Reiswachs; Carnaubawachs, Candelillawachs, Ouricurywachs, Alfawachs, Korkfaserwachs, Zuckerrohrwachs, Japanwachs und Sumachwachs; Montanwachs, mikrokristallinen Wachsen, Paraffinen und Ozokerit; Polyethylenwachsen, durch Fischer-Tropsch-Synthese hergestellten Wachsen; wachsartigen Copolymeren sowie deren Estern; Wachsen, die durch katalytische Hydrierung von tierischen Ölen oder pflanzlichen Ölen mit linearen oder verzweigten C₈₋₃₂-Fettketten hergestellt werden; hydriertem Jojobaöl, hydriertem Sonnenblumenöl, hydriertem Ricinusöl, hydriertem Kopraöl, hydriertem Lanolinöl; Siliconwachsen; und deren Gemischen ausgewählt ist.

6. Zusammensetzung nach einem der vorhergehenden Ansprüche, **dadurch gekennzeichnet, dass** das Wachs in einem Trockensubstanzgehalt von 0,1 bis 50 Gew.-%, bezogen auf das Gesamtgewicht der Zusammensetzung, vorzugsweise im Bereich von 0,5 bis 30 Gew.-% und besser im Bereich von 1 bis 20 Gew.-% enthalten ist.

7. Zusammensetzung nach einem der vorhergehenden Ansprüche, **dadurch gekennzeichnet, dass** die Partikel der Wachsdispersion ferner ölige und/oder pastöse Additive auf Fettbasis und/oder einen fettlöslichen Hilfsstoff/Wirkstoff enthalten.

8. Zusammensetzung nach einem der vorhergehenden Ansprüche, die ferner mindestens einen grenzflächenaktiven Stoff enthält.

9. Zusammensetzung nach einem der vorhergehenden Ansprüche, **dadurch gekennzeichnet, dass** das filmbildende Polymer unter den durch radikalische Polymerisation hergestellten Polymeren, Polykondensaten, Polymeren natürlicher Herkunft und deren Gemischen ausgewählt ist.

10. Zusammensetzung nach einem der vorhergehenden Ansprüche, **dadurch gekennzeichnet, dass** das filmbildende Polymer unter den Vinylpolymeren ausgewählt ist, die bei der Polymerisation von Monomeren gebildet werden, die unter den α,β-ethylenisch ungesättigten Carbonsäuren, den Estern dieser Säuren, den Amiden dieser Säuren, Vinylestern und Styrolmonomeren ausgewählt sind.

11. Zusammensetzung nach einem der vorhergehenden Ansprüche, **dadurch gekennzeichnet, dass** das filmbildende Polymer unter den Polyurethanen, Polyestern, Polyesteramiden, Polyamiden, Epoxyesterharzen und Polyharnstoffen ausgewählt ist.

12. Zusammensetzung nach einem der vorhergehenden Ansprüche, **dadurch gekennzeichnet, dass** das fïlmbildende Polymer unter Schellack, Sandarak, Dammarharzen, Elemi, Copalen, in Wasser unlöslichen Cellulosepolymeren und deren Gemischen ausgewählt ist.

13. Zusammensetzung nach einem der vorhergehenden Ansprüche, **dadurch gekennzeichnet, dass** das filmbildende Polymer in Form von in der wässrigen Phase dispergierten, festen Partikeln vorliegt.

14. Zusammensetzung nach einem der vorhergehenden Ansprüche, **dadurch gekennzeichnet, dass** das fïlmbildende Polymer in einer Trockensubstanzmenge von 0,1 bis 60 Gew.-%, bezogen auf das Gesamtgewicht der Zusammensetzung, vorzugsweise 0,5 bis 40 Gew.-% und besser 1, bis 30 Gew.-% enthalten ist.

15. Zusammensetzung nach einem der vorhergehenden Ansprüche, **dadurch gekennzeichnet, dass** der wasserlösliche Ester einer aromatischen Säure bei der Veresterung einer aromatischen Säure mit mindestens einer Hydroxygruppe einer hydroxylierten Verbindung gebildet wird, die unter den Dimethiconcopolyolen, Polyethylenglycol/Polypropylenglycol-Blockpolymeren und polyalkoxylierten Methylglycosiden ausgewählt ist.

16. Zusammensetzung nach einem der vorhergehenden Ansprüche, **dadurch gekennzeichnet, dass** die aromatische Säure unter Benzoesäure, Phenylessigsäure, Zimtsäure, 3-Phenylpropansäure, Salicylsäure, Terephthalsäure, Trimellitsäure und Pyromellitsäure ausgewählt ist.

17. Zusammensetzung nach einem der vorhergehenden Ansprüche, **dadurch gekennzeichnet, dass** die aromatische Säure die Benzoesäure ist.

18. Zusammensetzung nach einem der vorhergehenden Ansprüche, **dadurch gekennzeichnet, dass** der Ester der aromatischen Säure ein Dimethiconcopolyolbenzoat ist.

19. Zusammensetzung nach Anspruch 15, **dadurch gekennzeichnet, dass** das polyalkoxylierte Methylglucosid ein ethoxyliertes oder propoxyliertes Methylglucosid ist.

20. Zusammensetzung nach einem der Ansprüche 1 bis 17, **dadurch gekennzeichnet, dass** der Ester der aromatischen Säure ein ethoxyliertes oder propoxyliertes Methylglucosidbenzoat ist.

21. Zusammensetzung nach Anspruch 20, **dadurch gekennzeichnet, dass** der wasserlösliche Ester einer aromatischen Säure ein ethoxyliertes Methylglucosidbenzoat der folgenden Formel (I) ist: 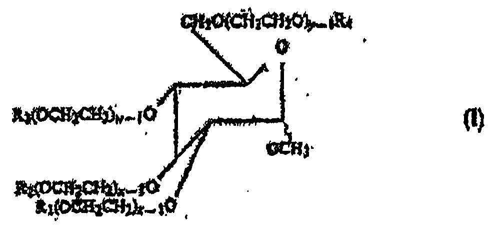 worin
- R₁, R₂, R₃ und R₄ 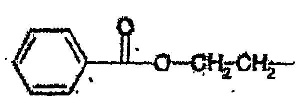 bedeuten
und w + x + y + z im Bereich von 10 bis 20 liegt.

22. Zusammensetzung nach Anspruch 20, **dadurch gekennzeichnet, dass** der Ester der aromatischen Säure ein propoxyliertes Methylglucosidbenzoat der folgenden Formel (II) ist: 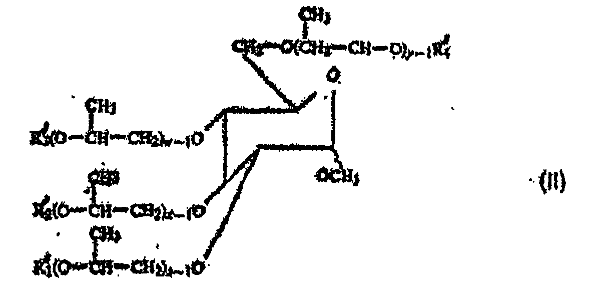 worin
- R'₁, R'₂, R'₃, R'₄ 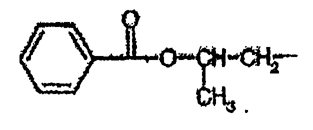 bedeuten
und w + x + y + z im Bereich von 10 bis 20 liegt.

23. Zusammensetzung nach einem der Ansprüche 1 bis 17, **dadurch gekennzeichnet, dass** der Ester der aromatischen Säure bei der Veresterung einer aromatischen Säure mit einem Polyethylenglycol/Polypropylenglycol-Blockpolymer gebildet wird.

24. Zusammensetzung nach Anspruch 23, **dadurch gekennzeichnet, dass** das Polyethylenglycol/Polypropylenglycol-Blockpolymer unter den Dreiblock-Polykondensaten Polyethylenglycol/Polypropylenglycol/Polyethylenglycol oder Polypropylenglycol/Polyethylenglycol/Polypropylenglycol ausgewählt ist.

25. Zusammensetzung nach Anspruch 23 oder 24, **dadurch gekennzeichnet, dass** der Ester der aromatischen Säure ein Benzoat eines Polyethylenglycol/Polypropylenglycol-Blockcopolymers ist.

26. Zusammensetzung nach Anspruch 25, **dadurch gekennzeichnet, dass** der Ester der aromatischen Säure der folgenden Formel (III) entspricht:
R⁵-CO-O-(-CH₂-CH₂-O)ₓ-(-CH₂-CH(CH₃)-O)_{y}-(CH₂-CH₂-O)ₓ-O-CO-R'⁵ (III)
worin bedeuten:
- R⁵ und R'⁵ unabhängig voneinander ein Wasserstoffatom oder Phenyl, wobei mindestens eine der Gruppen R⁵ und R'⁵ eine Phenylgruppe bedeutet,
- x und y unabhängig voneinander eine Zahl im Bereich von 2 bis 100.

27. Zusammensetzung nach Anspruch 25, **dadurch gekennzeichnet, dass** der Ester der aromatischen Säure der folgenden Formel (IV) entspricht:
R⁵-CO-O-(-CH(CH₃)-CH₂-O)ₓ-(-CH₂-CH₂-O)_{y}-(CH₂-CH(CH₃)-O)ₓ-O-CO-R'⁵ (IV)
worin bedeuten:
- R⁵ und R'⁵ unabhängig voneinander ein Wasserstoffatom oder Phenyl, wobei mindestens eine der Gruppen R⁵ und R'⁵ eine Phenylgruppe bedeutet,
- x und y unabhängig voneinander eine Zahl im Bereich von 2 bis 100.

28. Zusammensetzung nach Anspruch 26 oder 27, **dadurch gekennzeichnet, dass** x und y eine Zahl von 2 bis 30 bedeuten.

29. Zusammensetzung nach einem der vorhergehenden Ansprüche, **dadurch gekennzeichnet, dass** der wasserlösliche Ester der aromatischen Säure in der erfindungsgemäßen Zusammensetzung in einer Menge von 0,1 bis 20 Gew.-%, bezogen auf das Gesamtgewicht der Zusammensetzung, vorzugsweise 1 bis 15 Gew.-% und besser 5 bis 15 Gew.-% enthalten ist.

30. Zusammensetzung nach einem der vorhergehenden Ansprüche, **dadurch gekennzeichnet, dass** das filmbildende Polymer und der Ester der aromatischen Säure in einem Gewichtsverhältnis filmbildendes Polymer/Ester der aromatischen Säure im Bereich von 0,1 bis 3 und vorzugsweise 0,5 bis 2,5 enthalten sind.

31. Zusammensetzung nach einem der vorhergehenden Ansprüche, **dadurch gekennzeichnet, dass** die Zusammensetzung ferner mindestens einen Zusatzstoff enthält, der unter den Verdickungsmitteln, Weichmachern, Koaleszenzmitteln, Füllstoffen, Farbmitteln, Wachsen, grenzflächenaktiven Stoffen, Konservierungsmitteln, Ölen und Parfums ausgewählt ist.

32. Zusammensetzung nach einem der vorhergehenden Ansprüche, **dadurch gekennzeichnet, dass** die Zusammensetzung eine kosmetische Zusammensetzung ist.

33. Zusammensetzung nach Anspruch 32, **dadurch gekennzeichnet, dass** sie als Eyeliner, Lidschatten, Produkt zum Schminken des Körpers, Produkt für die Lippen, Mascara, Nagellack, Make-up, Produkt für die Augenbrauen, Produkt für die Haarbehandlung oder Produkt für die Pflege der Haut vorliegt.

34. Kosmetisches Verfahren zur nicht therapeutischen Behandlung oder zum Schminken von Keratinsubstanzen, das das Aufbringen einer Zusammensetzung nach einem der Ansprüche 1 bis 33 auf die Keratinsubstanzen umfasst.

35. Verfahren nach Anspruch 34, **dadurch gekennzeichnet, dass** es sich bei den Keratinsubstanzen um die Haut handelt.

36. Verwendung einer Zusammensetzung nach einem der Ansprüche 1 bis 33, um einen glänzenden und/oder nicht klebrigen Film auf den Keratinsubstanzen abzuscheiden.

37. Verwendung eines Esters einer aromatischen Säure, der bei Umgebungstemperatur flüssig und in Wasser löslich ist, eines filmbildenden Polymers und einer wässrigen Wachsmikrodispersion in einer kosmetischen Zusammensetzung, die ein wässriges Medium enthält, um eine Zusammensetzung herzustellen, die sich auf den Keratinsubstanzen und insbesondere der Haut einfach aufbringen und/oder dort leicht verteilen lässt, und/oder um auf den Keratinsubstanzen und insbesondere auf der Haut einen glänzenden und/oder nicht klebrigen Film abzuscheiden.
